# EUROPEAN PATENT SPECIFICATION

(11) **EP 2 670 435 B1**
(45) Date of publication and mention of the grant of the patent: **23.08.2017**
(21) Application number: 12741908.3
(22) Date of filing: 18.01.2012
(51) Int. Cl.: A61K 39/395, A61P 35/00, C07K 16/18, C07K 16/30, G01N 33/574

(54) **USE OF ANTI-DKK1 MONOCLONAL ANTIBODY FOR TREATMENT OF LIVER CANCER**
VERWENDUNG MONOKLONALER ANTIKÖRPER GEGEN DKK1 ZUR BEHANDLUNG VON LEBERKREBS
UTILISATION D'UN ANTICORPS MONOCLONAL ANTI-DKK1 POUR LE TRAITEMENT DU CANCER DU FOIE

(30) Priority: 01.02.2011 US 201161438357 P
(43) Date of publication of application: 11.12.2013
(73) Proprietor: The University of Hong Kong, Hong Kong (CN)
(72) Inventor: NG LUI, Oilin, Irene, Hong Kong (CN); TUNG, Kwokkwan, Kwai Chung, NT Hong Kong (CN)
(74) Representative: Isarpatent
(86) International application number: PCT/CN2012/070542
(87) International publication number: WO 2012/103787

(56) References cited:
- WO-A1-2008/064570
- WO-A2-2007/084344
- CN-A- 101 273 144
- CN-A- 101 398 433
- CN-A- 101 400 406
- QIN ET AL: "Proliferation and migration mediated by Dkk-1/Wnt/beta-catenin cascade in a model of hepatocellular carcinoma cells", TRANSLATIONAL RESEARCH, ELSEVIER, AMSTERDAM, NL, vol. 150, no. 5, 25 October 2007 (2007-10-25), pages 281-294, XP022380216, ISSN: 1931-5244, DOI: 10.1016/J.TRSL.2007.06.005
- YU B ET AL: "Elevated expression of DKK1 is associated with cytoplasmic/nuclear beta-catenin accumulation and poor prognosis in hepatocellular carcinomas", JOURNAL OF HEPATOLOGY, ELSEVIER, AMSTERDAM, NL, vol. 50, no. 5, 1 May 2009 (2009-05-01), pages 948-957, XP026044591, ISSN: 0168-8278, DOI: 10.1016/J.JHEP.2008.11.020 [retrieved on 2009-01-09]

## Description

### TECHNICAL FIELD

This disclosure relates generally to treating hepatocellular carcinoma by the use of anti-DKK1 monoclonal antibody.

### BACKGROUND

Hepatocellular carcinoma (HCC) is the sixth most common cancer worldwide (Parkin, Bray et al., J. Clin 55:74-1080 (2005) and particularly prevalent in Southeast Asia and China.

The current molecular targets for treatment of HCC are restricted to several cell signaling pathways, like EGFR, IGF-R1, PI3K/AKT/mTOR pathway, Ras/RAF/MAPK, VEGF and VEGFR pathways etc. Our invention suggests that other signaling pathway(s), like Wnt signaling pathway, may also be a potential therapeutic target in liver cancer.

Wnt/β-catenin pathway is one of the important signaling pathways and a potential therapeutic target in liver cancer (Breuhahn, Longerich et al., Oncogene 25: 3787-380 (2006); Greten, Korangy et al., Nature 391: 357-362 (2009). Dickkopf-1 (DKK1) is a secreted glycoprotein identified as a novel gene that functions as head inducers in Xenopus embryo (Glinka, Wu et al., Nature 391: 357-362 (1998); Fedi, Bafico et al., J. Biol Chem 274: 19465-19472 (1999). It acts as a Wnt inhibitor by binding to and antagonizing Wnt coreceptor LRP5/6 and blocks the interaction between Wnt ligands and Frizzled receptors (Bafico, Liu et al., Nat Cell Biol 3: 683-686 (2001); Mao, Wu et al., Nature 411: 321-328 (2001); Semenov, Tamai et al., Curr Biol: 11:951-961 (2001). Interestingly, although DKK1 is a Wnt inhibitor, its functional role in cancers is controversial. It is a potential tumor suppressor in some cancers, like medulloblastoma, melanoma and choriocarcinoma (Kuphal, Lodermeyer et al., Oncogene 25:5027-5036 (2006); Vibhakar, Foltz et al., Neuro Oncol 9: 135-144 (2007). DKK1 level has also been reported to decrease during the progression of prostate cancer from primary tumor to metastasis (Hall, Daignault et al., Prostate 68: 3696-1404 (2008). However, it has recently been found to be upregulated in various cancers including breast, lung, ovarian, prostate cancers and HCC (Forget, Turcotte et al., BrJ Cancer 96: 646-653 (2007); Hall, Daignault et al. 2008; Sheng, Huang et al., Clin Chem 55: 1656-664 2009; Shizhuo, Tao et al., Int J Biol Markers 24: 165-170 (2009); Yu, Yang et al., J. Hepatol 50: 948-957 (2009). It appears that the functional role of DKK1 may be different in different cancer types.

DKK1 is a small secretory protein with 266-amino acid (35 kDa) and can be secreted into blood stream. In fact, serum DKK1 has been found to be increased in patients with cancers of various types and is a useful biomarker (Politou, Heath et al., Int J Cance 119: 1728-1731 (2006); Qian, Xie et al., Blood 110: 1587-1594 (2007); Heider, Kaiser et al., Eur J Haematol 82: 31-38 (2009); Sato, Yamabuki et al., Cancer Res. (2010). It has also been found to be a prognostic marker and predicts clinical outcome in esophageal, ovarian and lung carcinomas (Yamabuki, Takano et al., Cancer Res 67: 2517-2525 (2007); Makino, Yamasaki et al., Ann Surg Oncol 16: 2058-2064 (2009); Sheng, Huang et al., Clin Chem 55: 1656-1664 (2009); Shizhuo, Tao et al. 2009). However, the clinical and prognostic significance of serum DKK1 in HCC patients remains to be determined. Concerning HCC there are only a few molecular targeted therapies including monoclonal antibodies and more molecular inhibitors. There is a need for the application of anti-DKK1 monoclonal antibody against liver cancer expressing higher levels of DKK1.

D1 QIN et al., TRANSLATIONAL RESEARCH, vol. 150, no. 5, pages 281-294, disclose the use of siRNA targeting DKK1 in proliferation and migration of HCC and its effect on Wnt/beta-catenin. The authors discuss the contribution of DKK1/ Wnt/beta-catenin cascade to the metastatis of hepatoma cells.

WO 2007/084344 A2 shows in examples 3 and 4 that anti-DKK1 antibodies inhibit the Wnt antagonistic activity of DKK1. HCC cell lines have not been used.

Yu et al., Journal of Hepatology, vol. 50, pages 948-957, describe the detection of high levels of DKK1 protein in some serologic samples from 128 HCC patients.

WO 2008/064570 A1 discusses in example 14 the use of an anti-GEP antibody for the treatment of HCC. An anti-DKK1 antibody is not described to be used in the treatment of HCC.

### SUMMARY

The present invention provides an anti-DKK1 monoclonal antibody for use in a method of treating and preventing DKK1-mediated hepatocellular carcinoma. In addition, the use of anti-DKK1 antibody not only protects against liver cancer, it suppresses liver cancer progression, prevents tumor metastasis and reduces tumor recurrence. Specifically, an anti-DKK1 monoclonal antibody for use in a method of treating or preventing DKK1-mediated hepatocellular carcinoma is described, wherein the method comprises administering to a subject having hepatocellular carcinoma and (a) high levels of DKK1 protein in serum of the subject compared to subjects without hepatocellular cancer, or (b) overexpression of DKK1 in hepatocellular carcinoma tissue of the subject compared to non-tumorous hepatocellular tissue of the subject an effective amount of an anti-DKK1 monoclonal antibody in a pharmaceutical formulation.

Another aspect described but not claimed herein is to use DKK1 as a biomarker for tumor recurrence.

Another aspect described but not claimed herein is to detect the overexpression of DKK1 transcripts to determine the presence of liver cancer in a patient.

Another embodiment of the present invention is the use of anti-DKK1 monoclonal antibody to prevent liver cancer mediated by DKK1.

Another aspect described but not claimed herein is detecting the expression of DKK1 transcripts in paired HCC and non-tumorous tissue from the same patient, and if there is overexpression, administering anti DKK1 monoclonal antibody to the patient.

Another embodiment of the present invention is the use of anti-DKK1 monoclonal antibody to reduce DKK1-mediated tumor growth.

Another aspect described but not claimed herein is a DKK1 ELISA kit used in determining the serum DKK1 protein.

### BRIEF DESCRIPTION OF THE DRAWINGS

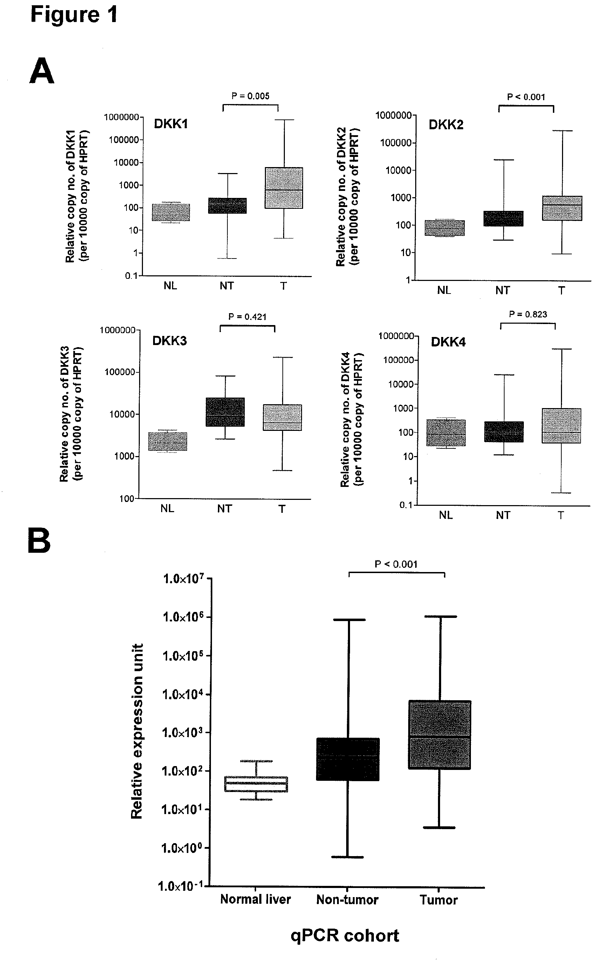

Figure 1 indicates transcript levels of DKK1 were upregulated in human HCC samples in both LDA and qPCR analysis. (A) A small-scale screening of different Wnt-related transcripts by quantitative-PCR-based LDA on 38 pairs of HCC samples showed significant upregulation of DKK1 and DKK2 transcripts but not DKK3 and DKK4 in the DKK family. (B) Real-time qPCR was done in the 99 paired HCC samples to validate the results obtained from LDA analysis. We found significant upregulation of DKK1 transcript levels in HCCs as compared with the non-tumorous livers (P <0.001). Ten cases of normal livers were also included for analysis.

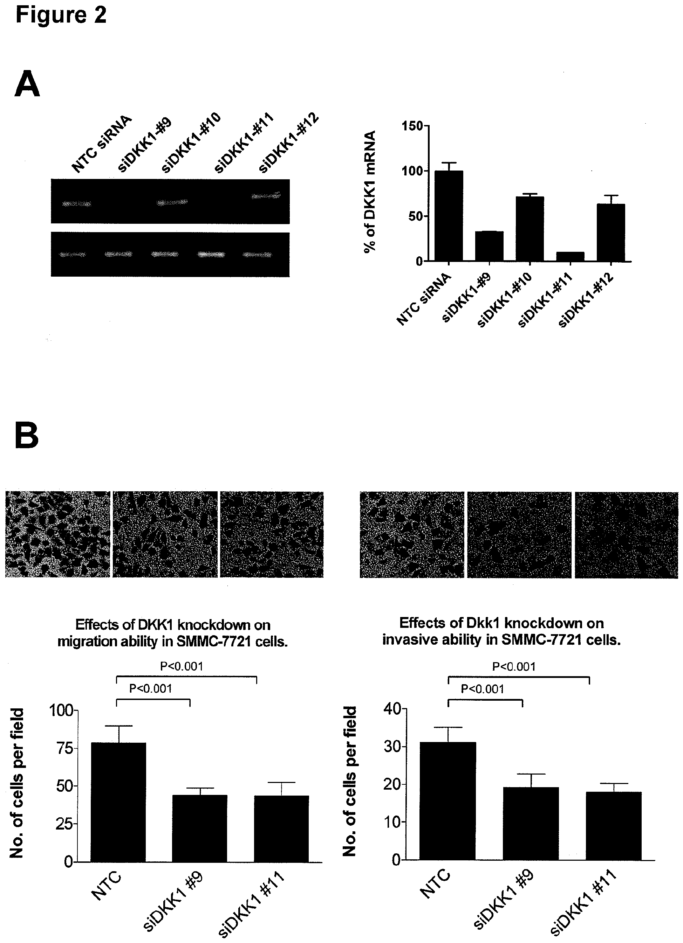

Figure 2 indicates knockdown of DKK1 reduced the migratory and invasive ability of SMMC-7721 cells. (A) siRNAs effectively knocked down the DKK1 transcript level in SMMC-7721 cells, which showed a relatively high level of endogenous secreted DKK1 as determined by qPCR and ELISA assay. The knockdown efficiency of siDKK1#11 and siDKK1#9 was 90% and 67%, respectively, and these clones were used in subsequent experiments. (B) Cell migration and invasion assays were performed in stable SMMC-7721 DKK1-knockdown clones. Knockdown of DKK1 with siRNAs significantly reduced the ability of the cells to migrate and invade.

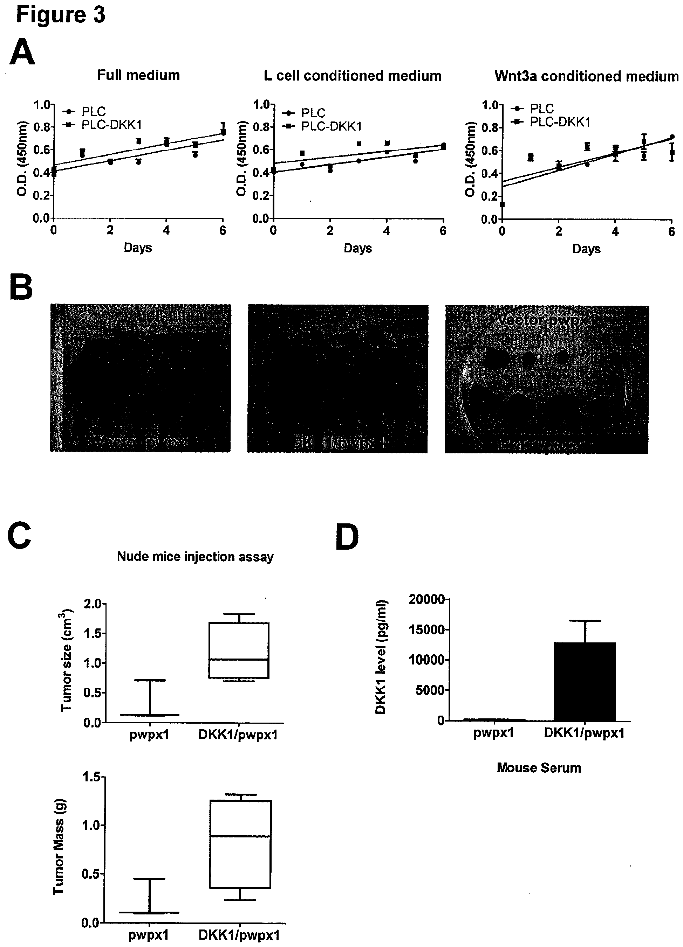

Figure 3 indicates DKK1 enhanced the tumor growth in vivo. (A) DKK1 did not affect the cell proliferation rate of HCC cells in vivo. Ectopic expression of DKK1 in PLC/PRF/5 resulted in no significant effect on the cell proliferation rate as assessed by MTT assay, either in the absence or presence of Wnt3a. (B) Nude mice injection assay was done by injecting DKK1-expressing PLC/PRF/5 cells into the right flank of the mice subcutaneously. (C) Size and mass of the tumors formed. The diameters of the tumors were measured 3 weeks after the injection and weighted after sacrifice. Tumors formed by DKK1-expressing cells grew larger than those of the vector control clones. (D) The DKK1 serum levels of the mice were detected using ELISA assay. High level of DKK1 was detected in mice with tumor formed by DKK1-expressing PLC/PRF/5 cells but the level was undetectable in mice with tumor formed by the control PLC/PRF/5 cells.

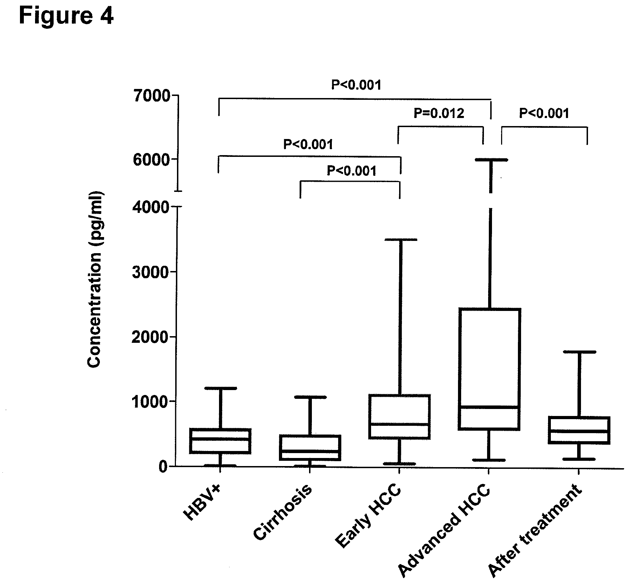

Figure 4 indicates a stepwise increase of serum protein levels of DKK1. A stepwise increase of the DKK1 serum levels was seen in HCC patients. The serum levels of DKK1 increased from HBV carriers and patients with cirrhosis, via early HCC to advanced HCC. The DKK1 serum levels in HCC patients after treatment were significantly lower (P <0.001).

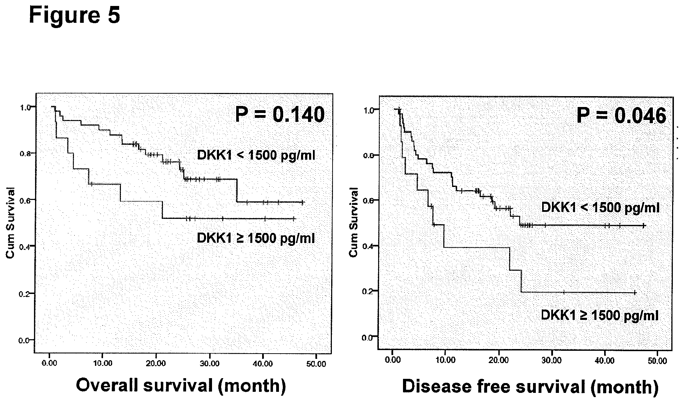

Figure 5 indicates higher levels of DKK1 were associated with shorter survival rate in HCC patients. Overall survival and disease-free survival rates in patients of the serum cohort. 1500 pg/ml was used as a cutoff point for the serum data.

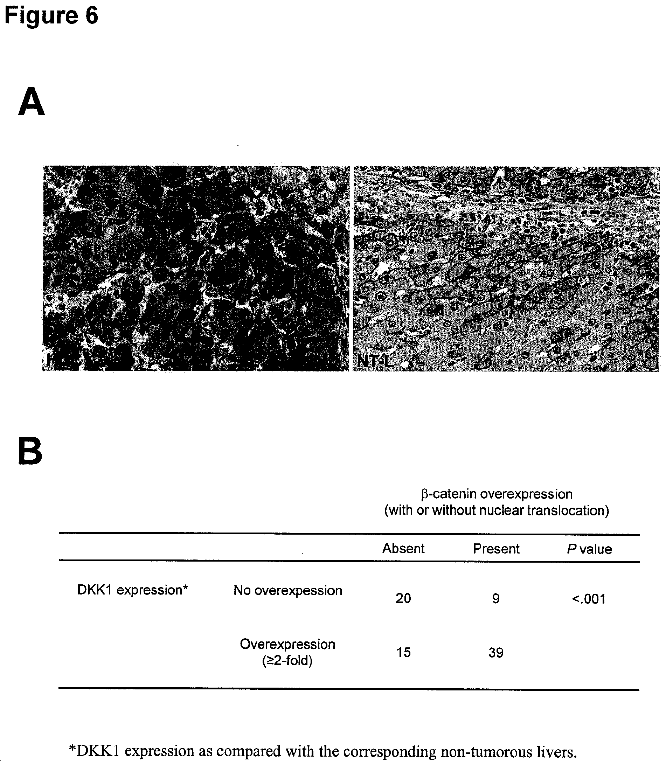

Figure 6 indicates the relationship between Wnt/β-catenin activation and upregulation of DKK1 level in HCC cells and human HCCs. (A) A representative case with β-catenin nuclear and cytoplasmic positivity. NT-L, nontumorous liver. (B) Statistical correlation between DKK1 transcript levels and β-catenin protein expression by immunohistochemistry staining. There was a significant association between DKK1 transcript levels and β-catenin cytoplasmic overexpression with or without nuclear translocation (P <0.001).

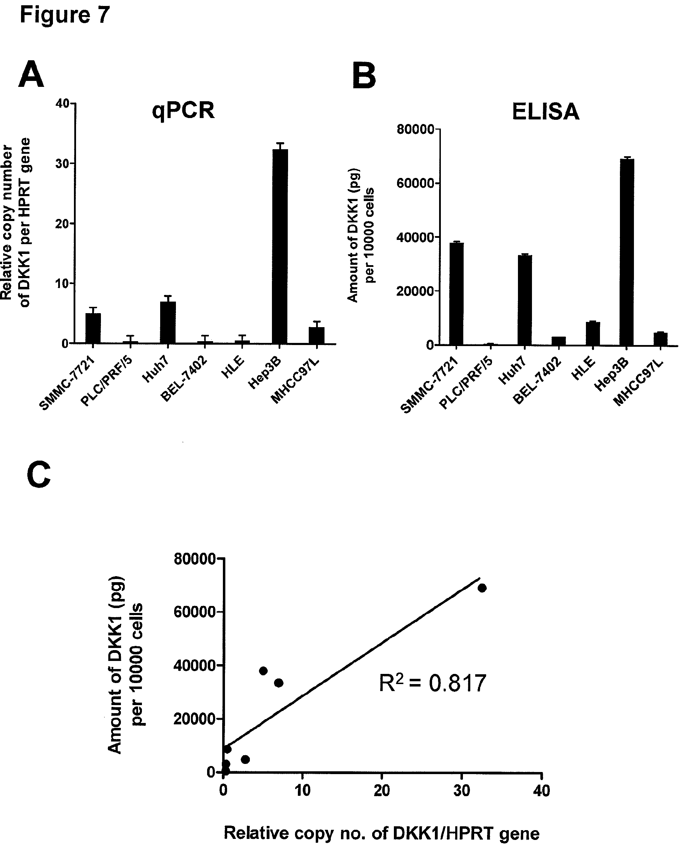

Figure 7 (A & B) indicates secreted levels of DKK1 in the culture media of 8 HCC cell lines. They largely corresponded to their transcript levels by qPCR. (C) X-Y plot showed the level of transcript and secreted protein level. Among HCC cell lines, transcript level was significant correlated the secreted protein level of DKK1 in culture medium.

### DETAILED DESCRIPTION

The present disclosure describes anti-DKK1 monoclonal antibody against serum and tissue DKK1 thereby treating liver cancer and preventing tumor metastasis and tumor recurrence. The use of the anti-DKK1 monoclonal antibody is based on the discovery of the upregulation of DKK1 in the serum and tissue of human liver cancer patients and its role on cell migration, invasion and tumor growth. Thus, DKK1 can be the therapeutic target for the prevention, amelioration and treatment of liver cancer, cancer metastasis and tumor recurrence. The use of anti-DKK1 monoclonal antibody enhances the survival rate of cancer patients with high levels of DKK1.

Several experiments were conducted to define the significance DKK1 on cell migration and invasion, and tumor growth and to define the use of anti-DKK1 monoclonal antibody against it.

High-throughput q-PCR analysis using the LDA analysis (Taqman® Low Density Array, LDA) was performed while studying the gene expression patterns of Wnt-signaling molecules on 38 pairs of human HCCs and their corresponding non-tumorous livers. From the data, two DKK genes, DKK1 and DKK2, were found to be frequently (65.8%, 25/38 and 60.5%, 23/38, respectively) overexpressed in human HCCs when compared with their corresponding non-tumorous livers (P = 0.005 and <0.001, respectively). There was no significant difference in the DKK3 and DKK4 transcript levels each between the tumors and their corresponding non-tumorous livers (P = 0.421 and 0.823, respectively). The expression levels of DKK1 transcript were analyzed in an independent cohorts, by qPCR analysis (n = 99 pairs). The DKK1 transcript in human HCCs was significantly upregulated as compared with the corresponding non-tumorous livers (P < 0.001). Overexpression of DKK1 (with a 2-fold cut-off) in human HCCs was frequent, being 60% in this cohort of patients we analyzed.

ELISA assay was analyzed to determine the serum DKK1 levels in a total of 241 patients, consisting of 50 HBV carriers without HCC (as control), 50 patients with cirrhosis without HCC, 50 patients with early HCC, 50 patients with advanced HCC, and 41 HCC patients after treatment. The serum DKK1 level in the HBV carrier group (mean ± S.D. = 431 ± 280 pg/ml) was not significantly different from that of the cirrhosis group (mean ± S.D. = 327 ± 274 pg/ml) (P = 0.086). In contrast, there was a stepwise increase in the serum DKK1 level from the cirrhosis group via the early HCC to advanced HCC group. The serum DKK1 level was significantly higher in patients with early HCC (mean ± S.D. = 881 ± 679 pg/ml) and advanced HCC (mean ± S.D. = 1498 ± 1280 pg/ml), as compared with the HBV carrier and cirrhosis groups (P < 0.001 for both). The serum DKK1 level was also significantly higher in the advanced HCC group than the early HCC one (P < 0.012). Of note, the serum DKK1 level was significantly lower (mean ± S.D. = 634 ± 366 pg/ml) in the group of HCC patients after treatment as compared with advanced HCC group (P < 0.001).

Serum levels of DKK1 had prognostic significance in HCC patients. It was found that serum levels of DKK1 correlated with patients' survival rates. A higher level of serum DKK1 level significantly correlated with shorter disease-free survival (P = 0.046). Besides, patients with high serum DKK1 showed a trend of poorer overall survival, although it did not reach statistical significance (P = 0.140). A high level of serum DKK1 (>1500 pg/ml) was significantly associated with a larger tumor size (P = 0.027), signifying an increased tumor burden producing more DKK1 secretion into the blood stream. It was observed that patients with advanced tumor stage tended to have a higher level of DKK1, although it did not reach statistical significance (P = 0.075). There was, however, no significant correlation between serum DKK1 and serum alpha-fetoprotein (APF) levels (P = 0.634).

It was determined whether DKK1 had effects on HCC cell mobility and invasive ability. siRNAs were utilized to knock down DKK1 in SMMC-7721 HCC cells, which had a relatively higher secreted DKK1 protein level among the HCC cell lines tested. The siRNAs effectively knocked down DKK1, with up to 85% reduction as confirmed by qPCR. Using transwell migration and Matrigel invasion assays, respectively, it was found that knockdown of DKK1 significantly reduced both migratory and invasive abilities of the two independent SMMC-7721 knockdown clones (P < 0.001 for both).

The effects of DKK1 on cell proliferation were investigated using the MTT assay. The Lentivirus-infected DKK1 stable clones were established in the PLC/PRF/5 HCC cell line, which had a low expression of DKK1 protein by ELISA assay. The proliferation rate of the DKK1-expressing cells was not significantly different from that of the vector control, either in the presence or absence of Wnt3a conditioned medium. As DKK1 is a secreted protein, it was hypothesized that DKK1 might alter the tumor microenvironment to promote tumor growth. To test this hypothesis, DKK1 expressing PLC/PRF/5 cells was injected into nude mice subcutaneously to observe its effect on tumorigenicity. The result showed that DKK1 expressing PLC/PRF/5 cells had a higher incidence of tumor formation and formed larger tumors in nude mice. Only mice injected with DKK1-expressing cells showed high level of serum DKK1 in their sera. The data indicates that DKK1 promotes tumor growth in vivo.

Upon noting the levels of DKK1, an anti-DKK1 monoclonal antibody is used to treat the cancer or reduce tumor growth in the liver. The monoclonal antibody can be administered in several dosage forms.

While it may be possible for an anti-DKK1 antibody to be administered as a raw composition, it is also possible to present them as a pharmaceutical formulation in order to be used in the presently described and claimed invention. Accordingly, a pharmaceutical formulation comprising an anti-DKK1 antibody or a pharmaceutically acceptable salt, ester, prodrug or solvate thereof, together with one or more pharmaceutically acceptable carriers thereof and optionally one or more other therapeutic ingredients is provided. The carrier(s) must be "acceptable" in the sense of being compatible with the other ingredients of the formulation and not deleterious to the recipient thereof. Proper formulation is dependent upon the route of administration chosen. Any of the well-known techniques, carriers, and excipients may be used as suitable and as understood in the art; e.g., in Remington's Pharmaceutical Sciences. The pharmaceutical compositions may be manufactured in a manner that is itself known, e.g., by means of conventional mixing, dissolving, granulating, dragee-making, levigating, emulsifying, encapsulating, entrapping or compression processes.

The formulations include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. All methods include the step of bringing into association a peptide or a pharmaceutically acceptable salt, ester, prodrug or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active peptide doses.

An anti-DKK1 antibody composition may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In addition to the formulations described previously, the monoclonal antibody composition may also be formulated as a depot preparation. Such long acting formulations may be administered by implantation (for example subcutaneously or intramuscularly) or by intramuscular injection. Thus, for example, the peptides may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the monoclonal antibody composition may take the form of tablets, lozenges, pastilles, or gels formulated in conventional manner. Such compositions may comprise the active ingredient in a flavored basis such as sucrose and acacia or tragacanth.

The composition may also be formulated in rectal compositions such as suppositories or retention enemas, e.g., containing conventional suppository bases such as cocoa butter, polyethylene glycol, or other glycerides.

Preferred unit dosage formulations are those containing an effective dose, as herein below recited, or an appropriate fraction thereof, of the monoclonal antibody.

The amount of the monoclonal antibody for a single dosage form above or in combination with carrier materials will vary depending upon the severity of the liver cancer and particular mode of administration. The phrase "the therapeutic use" is intended to qualify the amount of monoclonal antibody used in its treatment of liver cancer. This amount will be used to treat or reduce the tumor size for HCC tumors.

It should be understood that in addition to the ingredients particularly mentioned above, the formulations may include other agents conventional in the art having regard to the type of formulation in question, for example those suitable for oral administration may include flavoring agents.

The following examples show various defined uses of anti-DKK1 monoclonal antibody and the significance in qualitative measurement of DKK1.

### EXAMPLES

### Example 1.

### Effect of anti-DKK1 monoclonal antibody on tumor cell proliferation in vitro

To investigate the effect of anti-DKK1 monoclonal antibody on tumor cell proliferation *in vitro,* HCC cells (100 cells per well) with high level of DKK1 such as SMMC-7721, Hep3B and Huh7 were seeded into 96-well plates and incubated at 37°C overnight. The next day, 100 µl culture medium with or without 1µg/ml of anti-DKK1 monoclonal antibody was replaced in each well and incubated at 37°C for 24, 48, 72 and 96 hours. After the defined incubation time, cell proliferation assay (MTT) was done to measure the cell proliferation rate. Instead of MTT assay, the cell proliferation rate can be obtained by cell counting assay using cell counter. Our data showed that anti-DKK1 monoclonal antibody can suppress the proliferation of HCC cells.

### Example 2.

### Effect of anti- DKK1 monoclonal antibody on tumor cell migration and invasion in vitro

Cell migration assay was performed using transwell inserts with polycarbonate membranes of 8.0-µm pore size (Corning Inc., NY). Matrigel invasion assay was done using transwell insert pre-coated with Matrigel (BD Biosciences, San Jose, CA). In brief, 1 x 10⁵ cells in serum-free medium were seeded onto the upper chamber. Culture medium containing 10% FBS was used as a chemoattractant in the lower chamber. Anti-DKK1 monoclonal antibody (1, 5, 10µg/ml) was added into both the upper chamber and lower chamber of the transwells. Cells were then incubated in a humidified incubator at 37°C for 12 h (for migration assay) or 24 h (for invasion assay). Migrated or invaded cells on the lower surface of the membrane were then fixed with methanol, stained with crystal violet, photographed and counted in 5 random fields. Each experiment was performed thrice. The data showed that anti-DKK1 monoclonal antibody can suppress the cell migration and invasive ability of HCC cells.

### Example 3.

### Effect of anti-DKK1 monoclonal antibody on tumor formation in vivo

To assess the effect of anti-DKK1 monoclonal antibody on the formation or initiation of HCC tumor, HCC cells with high level of DKK1 such as SMMC-7721, Hep3B, Huh7 and MHCC97L/H were trypsinized and resuspended in phosphate-buffered saline. Cells (2 x 10⁶) were injected subcutaneously into the flank of 6-week-old male immune-deficient mice using a 25-gauge needle (n=6 for each group of experiments). Then, mice were injected with anti-DKK1 antibody or control IgG (200 µg per mouse) by intraperitoneal injection. The injection procedure was done as twice a week with a total of 10 injections for each group of mice. Tumor size was monitored weekly by measuring the largest and smallest diameters of tumor and estimated according to the formula: volume = 1 / 2 x (largest diameter) x (smallest diameter)². Our data showed that anti-DKK1 monoclonal antibody can suppress the formation of tumor *in vivo.*

### Example 4.

### Effect of anti-DKK1 monoclonal antibody on tumor growth in vivo

To assess the suppressive effect of anti-DKK1 monoclonal antibody on the growth of HCC tumor, HCC cells with high level of DKK1 such as SMMC-7721, Hep3B, Huh7 and MHCC97L/H were trypsinized and resuspended in phosphate-buffered saline. Cells (2 x 10⁶) were injected subcutaneously into the flank of 6-week-old male immune-deficient mice using a 25-gauge needle (n=12 for each group of experiments). When the xenografts grew to a size of around 5 mm diameter, the mice were divided randomly into 2 groups of six mice each. Mice were then injected with either anti-DKK1 antibody or control IgG (200 µg per mouse) by intraperitoneal injection. The injection procedure was done as twice a week with a total of 10 injections for each group of mice. Tumor size was monitored weekly by measuring the largest and smallest diameters of tumor and estimated according to the formula: volume = 1 / 2 x (largest diameter) x (smallest diameter)². The data showed that anti-DKK1 monoclonal antibody can suppress tumor growth *in vivo.*

### Example 5.

### Effect of anti-DKK1 monoclonal antibody on tumor metastasis in vivo

Orthotopic implantation was used as a mouse model to reveal the effect of anti-DKK1 monoclonal antibody on tumor metastasis. First, a left subcostal incision was made under anaesthesia with Pentobarbital (80mg/kg) by intra-peritoneal. The left lobe of the liver was exposed and then one or two pieces of tumor tissue (about 1 mm³ in size from subcutaneous injection tumors) were implanted into the liver tissue with forceps. Abdominal wall was closed and mice were kept in cages under specific-pathogen-free conditions. Analgesia was given for three days after the operation. The mice were kept for 4 weeks to allow the growth of tumors. Then, the mice were divided randomly into 2 groups of six mice each. Mice were then injected with either anti-DKK1 antibody or control IgG (200 µg per mouse) by intraperitoneal injection. The injection procedure was done as twice a week with a total of 10 injections for each group of mice. After 10 injections, mice were euthanatized by overdose of Pentobarbitol (150-200mg/kg by intra-peritoneal). Tissues include livers and lungs were harvested for immunohistochemistry to reveal the effect of anti-DKK1 antibody on tumor metastasis.

In addition, the tail vein injection or direct liver injection assays were also used as mouse model to reveal the effect of anti-DKK1 monoclonal antibody on tumor metastasis. Tumor cells (1 x 10⁶) were injected directly into tail vein or liver of immune-deficient mice (n=12 for each group of experiments). Mice were then injected with anti-DKK1 antibody or control IgG (200 µg per mouse) by intraperitoneal injection. The injection procedure was done as twice a week with a total of 10 injections for each group of mice. After 10 injection, mice were euthanatized by overdose of Pentobarbitol (150-200mg/kg by intra-peritoneal). Tissues include livers and lungs were harvested for immunohistochemistry to reveal the effect of anti-DKK1 antibody on tumor metastasis.

The data showed that anti-DKK1 monoclonal antibody can suppress tumor metastasis *in vivo.*

### Example 6.

The use of serum DKK1 protein in prognosis of cancer: Biomarker for tumor recurrence

From the data, it is shown that serum levels of DKK1 have prognostic significance in HCC patients. A higher level of serum DKK1 level significantly correlated with shorter disease-free survival and showed a trend of poorer overall survival. In addition, it was observed that patients with advanced tumor stage tended to have a higher level of DKK1. Therefore, measurement of the DKK1 level by DKK1 ELISA assay predicts tumor recurrence in HCC patients.

### Example 7.

### Antibodies of invention

The antibodies used in this invention are purchased from commercial sources. Any commercially available monoclonal or polyclonal antibodies against DKK1 protein are useful. They include antibodies from R&D Systems, Santa Cruz biotechnology, Novartis Oncology and Pfizer etc. In addition, the said antibodies in this invention can be obtained from any other sources. For example, antibodies can be in-house antibody that newly raised against human DKK1 protein. The anti-DKK1 antibody can be humanized antibody or not.

### Example 8.

### Patient samples

A cohort of 99 pairs of HCCs and their corresponding non-tumorous liver tissues were employed for real time-quantitative PCR (qPCR). All patients had surgical resection at Queen Mary Hospital, The University of Hong Kong and were randomly selected for this study. The resected specimens were obtained immediately after surgical resection, snap-frozen in liquid nitrogen, and kept at -80°C. A total of 241 serum samples from 50 patients with early HCC (TNM stages I & II), 50 patients with advanced HCC (TNM stages III & IV), 41 HCC patients after treatment for HCC (median time after treatment, 6 months), 50 patients with cirrhosis, and 50 hepatitis B virus (HBV) carriers collected at Queen Mary Hospital were evaluated. Serum samples were kept at -80°C prior to use. The characteristics of the 100 patients with early and advanced HCC of the serum cohort are summarized in Table 1.

### Example 9.

### qPCR and TaqMan low density array (LDA)

Total RNA was extracted from HCC cell lines and human HCC samples by TRIzol reagent (Invitrogen). First-strand cDNA was synthesized from 1 µg of total RNA using random hexamers with GeneAmp RNA PCR Kit (Applied Biosystems, Foster City, CA). The cDNA was then used for detecting the expression level of target genes with TaqMan Gene Expression Assays (Applied Biosystems). Pre-designed Taqman® low density custom arrays were used for LDA analysis. The Wnt-related genes selected for LDA analysis is listed in Supplementary Table 1. Both qPCR and LDA were performed in Applied Biosystems 7900HT Fast Real-Time System (Applied Biosystems). Experiments were done in triplicate for qPCR and duplicate for LDA. The expression of target genes in paired HCC and non-tumorous liver tissues from the same patient was expressed as T/NT ratio. A T/NT ratio >2 was defined as overexpression in tumor tissue. Upon the discovery of DKK1 transcript wherein the T/NT overexpression ratio is greater than 2, the patient in need thereof is administered anti-DKK1 monoclonal antibody.

### Example 10.

### ELISA assay

Serum DKK1 and secreted DKK1 from cell culture medium were measured by DueSet^{®} ELISA Development kit (R&D Systems, Minneapolis, MN, USA). Briefly, 96-well Maxisorp NUNC-Immuno plates (Thermo Fisher Scientific, Roskilde, Denmark) were coated overnight at room temperature with monoclonal mouse anti-human DKK1 capture antibody (R&D Systems) in 1 x PBS. Plates washed with wash buffer (1 x PBS with 0.05%Tween 20) was blocked with 1 x Assay Diluent (BioLegend, San Diego, CA) for 1 h at room temperature. Diluted patients' sera or conditioned media were incubated for 2 h. Plates were then washed with wash buffer. The goat anti-human detection antibody (R&D Systems) was added and incubated for another 2 h. Streptavidin-horseradish peroxidase was added and incubated for 20 min and after washing, TMB Substrate Reagent (BioLegend) was added for another 20 min. The substrate reaction was stopped by 2 N sulphuric acid and extinction was measured at 450 nm wavelength using Infinite F200 Tecan multiplate reader (Tecan Austria GmbH, Salzburg, Austria).

### Example 11.

### Cell lines

HCC cell lines PLC/PRF/5, Huh7 and Hep3B were purchased from the American Type Culture Collection (Manassas, VA), HLE purchased from the Japanese Cancer Resources Bank (Tokyo, Japan), and BEL-7402 and SMMC-7721 obtained from the Shanghai Institute of Cell Biology, and MHCC-97L from Liver Cancer Institute of Fudan University (Shanghai, China). All HCC cells were maintained in DMEM supplemented with 10% fetal bovine serum (FBS) and 100 unit/ml penicillin and streptomycin (Invitrogen, Gaithersburg, MD).

### Example 12.

### Establishment of DKK1 knockdown and overexpression stable clones

Small interfering RNA (siRNA) oligos for knocking down DKK1 were purchased from Thermo Scientific Dharmacon^{®} (Thermo Fisher Scientific, Lafayette, CO). ON-TARGETplus^{™} DKK1 siRNA (LQ-003843-01) was transfected into HCC cells by Lipofectamine 2000^{™} according to the manufacturer's protocol (Invitrogen). Non-target control (NTC) siRNA was used as control. The knockdown efficiency was then confirmed by qPCR as described above. The full-length human DKK1 gene was amplified from human placenta cDNA library and subcloned into pGEM-T vector and then subcloned into lenti expression vector pWPXL. Lentiviral particles with DKK1 gene were made according to manufacturer's protocol for infection of HCC PLC/PRF/5 cells. Infected cells were then sorted by GFP marker with flow cytometry.

### Example 13.

### In vitro cell migration and invasion assays

Cell migration assay was performed using transwell inserts with polycarbonate membranes of 8.0-µm pore size (Corning Inc., NY). Matrigel invasion assay was done using transwell insert pre-coated with Matrigel (BD Biosciences, San Jose, CA). In brief, 1 x 10⁵ cells in serum-free medium were seeded onto the upper chamber. Culture medium containing 10% FBS was used as a chemoattractant in the lower chamber. Cells were incubated in a humidified incubator at 37°C for 12 h (for migration assay) or 24 h (for invasion assay). Migrated or invaded cells on the lower surface of the membrane were then fixed with methanol, stained with crystal violet, photographed and counted in 5 random fields. Each experiment was performed thrice.

### Example 14.

### Cell proliferation assay (MTT)

Cell proliferation of DKK1 expressing cells was determined by CellTiter 96^{®} AQueous One solution cell proliferation assay (Promega, Madison, WI). Cells were seeded in 96-well plates and incubated at 37°C overnight. 100 µl culture medium was replaced in each well the next day and the assay was then performed according to the manufacturer's instruction. In brief, 15 µl of dye solution was added into each well and incubated for 30 min at 37°C. Absorbance was recorded at 595 nm using Infinite F200, Tecan multiplate reader (Tecan Austria GmbH). All experiments were performed in triplicate and in 2 independent experiments.

### Example 15.

### In vivo tumorigenicity assay

To assess the tumorigenicity, DKK1 stable cells were trypsinized and resuspended in phosphate-buffered saline. Cells (2 x 10⁶) were injected subcutaneously into the flank of 6-week-old male BALB/c nude mice using a 25-gauge needle (n=5 for each group of experiments). Tumor size was monitored weekly by measuring the largest and smallest diameters of tumor and estimated according to the formula: volume = 1 / 2 x (largest diameter) x (smallest diameter)².

### Example 16.

### Immunohistochemistry

Immunohistochemistry staining for β-catenin was performed on formalin-fixed, paraffin-embedded sections using the labeled streptavidin-biotin method. For antigen retrieval, the sections were immersed in buffer with 1 mM EDTA and boiled for 15 min. Anti-human β-catenin monoclonal antibody (BD Biosciences) was used at 1:100 dilution. For negative controls, the primary antibody was replaced with Tris-buffered saline. Nuclear positivity was assessed as either absent or present; cytoplasmic positivity as 0 (no staining) and 1 (positive staining); and membranous positivity as 0, 1 and 2.

### Statistical analysis

Clinicopathologic features of HCC patients included tumor size, cellular differentiation according to the Edmondson grading, venous invasion without differentiation into portal or hepatic venules, direct liver invasion, tumor microsatellite formation, tumor encapsulation, number of tumor nodules, and serum hepatitis B surface antigen (HBsAg) status were analyzed by SPSS for Windows 17.0 (SPSS Inc., Chicago, IL). Categorical data were analyzed by Chi square test or Fisher's exact test, whereas independent *t* or Mann-Whitney test was used for continuous data wherever appropriate. The survival curves were assessed by Kaplan-Meier method and the statistical difference between two groups was evaluated by log-rank test. Results from the migration and invasion assays were analyzed using Student's *t*-test. Tests were considered significant when the *P* value was less than 0.05.

In support of the forgoing, more detailed explanations of the various embodiments of the invention are disclosed.

### Materials and methods

### Patient samples

A cohort of 99 pairs of HCCs and their corresponding non-tumorous liver tissues were employed for real time-quantitative PCR (qPCR). All patients had surgical resection at Queen Mary Hospital, The University of Hong Kong and were randomly selected for this study. The resected specimens were obtained immediately after surgical resection, snap-frozen in liquid nitrogen, and kept at -80°C. A total of 241 serum samples from 50 patients with early HCC (TNM stages I & II), 50 patients with advanced HCC (TNM stages III & IV), 41 HCC patients after treatment for HCC (median time after treatment, 6 months), 50 patients with cirrhosis, and 50 hepatitis B virus (HBV) carriers collected at Queen Mary Hospital were evaluated. Serum samples were kept at -80°C prior to use. The characteristics of the 100 patients with early and advanced HCC of the serum cohort are summarized in Table 1.

### qPCR and TaqMan low density array (LDA)

Total RNA was extracted from HCC cell lines and human HCC samples by TRIzol reagent (Invitrogen). First-strand cDNA was synthesized from 1 µg of total RNA using random hexamers with GeneAmp RNA PCR Kit (Applied Biosystems, Foster City, CA). The cDNA was then used for detecting the expression level of target genes with TaqMan Gene Expression Assays (Applied Biosystems). Pre-designed Taqman® low density custom arrays were used for LDA analysis. The Wnt-related genes selected for LDA analysis are listed in Supplementary Table 1. Both qPCR and LDA were performed in Applied Biosystems 7900HT Fast Real-Time System (Applied Biosystems). Experiments were done in triplicate for qPCR and duplicate for LDA. The expression of target genes in paired HCC and non-tumorous liver tissues from the same patient was expressed as T/NT ratio. A T/NT ratio >2 was defined as overexpression in tumor tissue. Thus, the patient is administered anti-DKK1 monoclonal antibody for treatment of human HCC.

### ELISA assay

Serum DKK1 and secreted DKK1 from cell culture medium were measured by DueSet^{®} ELISA Development kit (R&D Systems, Minneapolis, MN, USA). Briefly, 96-well Maxisorp NUNC-Immuno plates (Thermo Fisher Scientific, Roskilde, Denmark) were coated overnight at room temperature with monoclonal mouse anti-human DKK1 capture antibody (R&D Systems) in 1 x PBS. Plates washed with wash buffer (1 x PBS with 0.05%Tween 20) was blocked with 1 x Assay Diluent (BioLegend, San Diego, CA) for 1 h at room temperature. Diluted patients' sera or conditioned media were incubated for 2 h. Plates were then washed with wash buffer. The goat anti-human detection antibody (R&D Systems) was added and incubated for another 2 h. Streptavidin-horseradish peroxidase was added and incubated for 20 min and after washing, TMB Substrate Reagent (BioLegend) was added for another 20 min. The substrate reaction was stopped by 2 N sulphuric acid and extinction was measured at 450 nm wavelength using Infinite F200 Tecan multiplate reader (Tecan Austria GmbH, Salzburg, Austria).

The present invention also includes a kit to determine the serum DKK1 protein. In one non-limiting example, the kit can include one or more reagents in containers as described above. In one container anti DKK1 monoclonal antibody is in a sealed container in the kit. The kit can have multiple containers arranged in the kit as desired.

### Cell lines

HCC cell lines PLC/PRF/5, Huh7 and Hep3B were purchased from the American Type Culture Collection (Manassas, VA), HLE purchased from the Japanese Cancer Resources Bank (Tokyo, Japan), and BEL-7402 and SMMC-7721 obtained from the Shanghai Institute of Cell Biology, and MHCC-97L from Liver Cancer Institute of Fudan University (Shanghai, China). All HCC cells were maintained in DMEM supplemented with 10% fetal bovine serum (FBS) and 100 unit/ml penicillin and streptomycin (Invitrogen, Gaithersburg, MD).

### Establishment of DKK1 knockdown and overexpression stable clones

Small interfering RNA (siRNA) oligos for knocking down DKK1 were purchased from Thermo Scientific Dharmacon^{®} (Thermo Fisher Scientific, Lafayette, CO). ON-TARGETplus^{™} DKK1 siRNA (LQ-003843-01) was transfected into HCC cells by Lipofectamine 2000^{™} according to the manufacturer's protocol (Invitrogen). Non-target control (NTC) siRNA was used as control. The knockdown efficiency was then confirmed by qPCR as described above. The full-length human DKK1 gene was amplified from human placenta cDNA library and subcloned into pGEM-T vector and then subcloned into lenti expression vector pWPXL. Lentiviral particles with DKK1 gene were made according to manufacturer's protocol for infection of HCC PLC/PRF/5 cells. Infected cells were then sorted by GFP marker with flow cytometry.

### In vitro cell migration and invasion assays

Cell migration assay was performed using transwell inserts with polycarbonate membranes of 8.0-µm pore size (Corning Inc., NY). Matrigel invasion assay was done using transwell insert pre-coated with Matrigel (BD Biosciences, San Jose, CA). In brief, 1 x 10⁵ cells in serum-free medium were seeded onto the upper chamber. Culture medium containing 10% FBS was used as a chemoattractant in the lower chamber. Cells were incubated in a humidified incubator at 37°C for 12 h (for migration assay) or 24 h (for invasion assay). Migrated or invaded cells on the lower surface of the membrane were then fixed with methanol, stained with crystal violet, photographed and counted in 5 random fields. Each experiment was performed thrice.

### Cell proliferation assay (MTT)

Cell proliferation of DKK1 expressing cells was determined by CellTiter 96^{®} AQueous One solution cell proliferation assay (Promega, Madison, WI). Cells were seeded in 96-well plates and incubated at 37°C overnight. 100 µl culture medium was replaced in each well the next day and the assay was then performed according to the manufacturer's instruction. In brief, 15 µl of dye solution was added into each well and incubated for 30 min at 37°C. Absorbance was recorded at 595 nm using Infinite F200, Tecan multiplate reader (Tecan Austria GmbH). All experiments were performed in triplicate and in 2 independent experiments.

### In vivo tumorigenicity assay

To assess the tumorigenicity, DKK1 stable cells were trypsinized and resuspended in phosphate-buffered saline. Cells (2 x 10⁶) were injected subcutaneously into the flank of 6-week-old male BALB/c nude mice using a 25-gauge needle (n=5 for each group of experiments). Tumor size was monitored weekly by measuring the largest and smallest diameters of tumor and estimated according to the formula: volume = 1/2 x (largest diameter) x (smallest diameter) 2.

### Immunohistochemistry

Immunohistochemistry staining for (3-catenin was performed on formalin-fixed, paraffin-embedded sections using the labeled streptavidin-biotin method. For antigen retrieval, the sections were immersed in buffer with 1 mM EDTA and boiled for 15 min. Anti-human (3-catenin monoclonal antibody (BD Biosciences) was used at 1:100 dilution. For negative controls, the primary antibody was replaced with Tris-buffered saline. Nuclear positivity was assessed as either absent or present; cytoplasmic positivity as 0 (no staining) and 1 (positive staining); and membranous positivity as 0, 1 and 2.

### Statistical analysis

Clinicopathologic features of HCC patients included tumor size, cellular differentiation according to the Edmondson grading, venous invasion without differentiation into portal or hepatic venules, direct liver invasion, tumor microsatellite formation, tumor encapsulation, number of tumor nodules, and serum hepatitis B surface antigen (HBsAg) status were analyzed by SPSS for Windows 17.0 (SPSS Inc., Chicago, IL). Categorical data were analyzed by Chi square test or Fisher's exact test, whereas independent *t* or Mann-Whitney test was used for continuous data wherever appropriate. The survival curves were assessed by Kaplan-Meier method and the statistical difference between two groups was evaluated by log-rank test. Results from the migration and invasion assays were analyzed using Student's *t*-test. Tests were considered significant when the *P* value was less than 0.05.

### RESULTS

### Transcript of DKK1 was significantly upregulated in human HCCs

A high-throughput q-PCR analysis was performed using the LDA analysis (Taqman® Low Density Array, LDA) to study the gene expression patterns of Wnt-signaling molecules (Supplementary Table 1) on 38 pairs of human HCCs and their corresponding non-tumorous livers. From our data, two DKK genes, DKK1 and DKK2, were found to be frequently (65.8%, 25/38 and 60.5%, 23/38, respectively) overexpressed in human HCCs when compared with their corresponding non-tumorous livers (*P* = 0.005 and <0.001, respectively, Mann-Whitney test) (Figure 1A). There was no significant difference in the DKK3 and DKK4 transcript levels each between the tumors and their corresponding non-tumorous livers (*P* = 0.421 and 0.823, respectively). In the present study, DKK1 was the focus of the investigation.

To confirm the results from LDA analysis that DKK1 was overexpressed in human HCCs and the expression levels of DKK1 transcript was analyzed in an independent cohorts, by qPCR analysis (n = 99 pairs). From the data, the DKK1 transcript in human HCCs was significantly upregulated as compared with the corresponding non-tumorous livers (*P* < 0.001, Mann-Whitney test) (Figure 1B). Overexpression of DKK1 (with a 2-fold cut-off) in human HCCs was frequent, being 60% in this cohort of patient analyzed. The overexpression of the DKK1 transcript indicates the need to administer the anti-DKK1 monoclonal antibody to the patient in need thereof.

### Suppression of DKK1 reduced both migration and invasive abilities of HCC cells

As DKK1 was overexpressed in human HCC, it was investigated to find how it affected tumor progression. DKK1 was found to be an inhibitor suppressing Wnt/β-catenin pathway and thus it was proposed to be a tumor suppressor gene [5,22]. However, the exact role of DKK1 during cancer progression and metastasis is controversial. Therefore, it was determined whether DKK1 had effects on HCC cell mobility and invasive ability. siRNAs were used to knock down DKK1 in SMMC-7721 HCC cells, which had a relatively higher secreted DKK1 protein level among the HCC cell lines tested (Figure 7). The siRNAs effectively knocked down DKK1, with up to 85% reduction as confirmed by qPCR (Figure 2A). Using transwell migration and Matrigel invasion assays, respectively, it was found that knockdown of DKK1 significantly reduced both migratory and invasive abilities of the two independent SMMC-7721 knockdown clones (*P* < 0.001 for both, Student's *t*-test) (Figure 2B).

### DKK1 enhanced growth of HCC cells in vivo

The effects of DKK1 on cell proliferation was investigated the MTT assay. Lentivirus-infected DKK1 stable clones were established in the PLC/PRF/5 HCC cell line, which had a low expression of DKK1 protein by ELISA assay (Figure 7B). The proliferation rate of the DKK1-expressing cells was not significantly different from that of the vector control (Figure 3A), either in the presence or absence of Wntβa conditioned medium. DKK1 expressing PLC/PRF/5 cells were injected into nude mice subcutaneously to observe its effect on tumorigenicity. The result showed that DKK1 expressing PLC/PRF/5 cells had a higher incidence of tumor formation and formed larger tumors in nude mice (Figure 3B&C). Only mice injected with DKK1-expressing cells showed high level of serum DKK1 in their sera (Figure 3D). The data indicate that DKK1 promotes tumor growth *in vivo.*

### Secreted DKK1 correlated with DKK1 transcript level in HCC cell lines

DKK1 is a small secretory protein with 266-amino acid (35 kDa). It was proven that DKK1 could be secreted from the tumors into the blood stream and patients with DKK1 overexpression in the tumors had increased protein levels of DKK1 in their blood. First, the efficacy of the ELISA assay in detecting human DKK1 protein was evaluated. As there were no previous data on the secreted DKK1 level in HCC cell culture or patient samples, 7 HCC cell lines to test for the presence and levels of DKK1 protein in culture media was employed. It was confirmed that DKK1 protein was detectable in the conditioned media after incubation (Figure 7B). Furthermore, the transcript levels of DKK1 by qPCR correlated well with the amount of the corresponding secreted DKK1 protein by ELISA in all the HCC cell lines tested as showed in Figure 7C (r² = 0.817, *P* = 0.005).

### Serum level of DKK1 was upregulated in HCC and showed a stepwise increase from cirrhosis to HCC

ELISA was then employed to determine the serum DKK1 levels in a total of 241 patients, consisting of 50 HBV carriers without HCC (as control), 50 patients with cirrhosis without HCC, 50 patients with early HCC, 50 patients with advanced HCC, and 41 HCC patients after treatment (Figure 4C). The serum DKK1 level in the HBV carrier group (mean ± S.D. = 431 ± 280 pg/ml) was not significantly different from that of the cirrhosis group (mean ± S.D. = 327 ± 274 pg/ml) (*P* = 0.086, Mann-Whitney test). In contrast, there was a stepwise increase in the serum DKK1 level from the cirrhosis group via the early HCC to advanced HCC group. The serum DKK1 level was significantly higher in patients with early HCC (mean ± S.D. = 881 ± 679 pg/ml) and advanced HCC (mean ± S.D. = 1498 ± 1280 pg/ml), as compared with the HBV carrier and cirrhosis groups (*P* < 0.001 for both, Mann-Whitney test). The serum DKK1 level was also significantly higher in the advanced HCC group than the early HCC one (*P* < 0.012, Mann-Whitney test). Of note, the serum DKK1 level was significantly lower (mean ± S.D. = 634 ± 366 pg/ml) in the group of HCC patients after treatment as compared with advanced HCC group (*P* < 0.001, Mann-Whitney test).

### Clinicopathologic correlation of serum DKK1 levels in HCC patients

The clinicopathologic characteristics of the patients for ELISA assay are summarized in Table 1. Upon clinicopathologic correlation, a high level of serum DKK1 (>1500 pg/ml) was significantly associated with a larger tumor size (*P* = 0.027, Fisher's exact test) (Table 2), signifying an increased tumor burden producing more DKK1 secretion into the blood stream. It was observed that patients with advanced tumor stage tended to have a higher level of DKK1, although it did not reach statistical significance (*P* = 0.075, Fisher's exact test). There was, however, no significant correlation between serum DKK1 and serum AFP levels (*P* = 0.634, Fisher's exact test).

### Serum level of DKK1 had prognostic significance in HCC patients

It was found that serum level of DKK1 correlated with patients' survival rates. A higher level of serum DKK1 level significantly correlated with shorter disease-free survival (*P* = 0.046, log-rank test) (Figure 5). Besides, patients with high serum DKK1 showed a trend of poorer overall survival, although it did not reach statistical significance (*P* = 0.140, log-rank test).

### Significant association between β-catenin overexpression and upregulation of DKK1 level in human HCCs

It has been suggested that DKK1 is a downstream target of Wnt/β-catenin signaling [23,24]. Therefore, we analyzed the Wnt/β-catenin protein expression by immunohistochemistry in 83 cases of human HCC and correlated it with overexpression of the corresponding DKK1 transcript levels. Wnt/β-catenin was overexpressed in the cytoplasm in 48 cases with or without nuclear staining (nuclear translocation). Nuclear staining was observed in 12 (14.5%) cases and all these cases with nuclear staining showed cytoplasmic staining. There was significant correlation between overexpression of DKK1 transcript levels (by >_2-fold) and (β-catenin cytoplasmic overexpression (*P* <0.001) with or without nuclear translocation (Figure 6).

### Discussion

DKK1 is believed to be a negative regulator of the Wnt/(β-catenin signaling pathway, but its role in cancers is controversial. It has recently been found to be upregulated in different cancers, including breast, lung, ovarian, prostate cancers and HCC [11,12,13,14,15]. From the high-throughput qPCR LDA analysis of Wnt-signaling molecules in human HCC samples, DKK1 was one of the genes showing overexpression. This was validated in an independent HCC cohort using qPCR analysis. Functionally, DKK1 played a crucial role in tumor cell migration and invasion. DKK1 showed enhanced tumor growth *in vivo* but did not promote cell proliferation *in vitro.* Stepwise increase in the DKK1 transcript levels in the multistep process of hepatocarcinogenesis, from chronic hepatitis and cirrhosis via early HCCs to advanced HCCs was documented.

Consistent with the findings of upregulated DKK1 transcript levels in HCCs, there is comprehensive evidence that HCC patients had upregulation of the serum DKK1. Not only was the upregulated serum DKK1 levels in HCC patients, it was documented for the first time a similar stepwise increase in the serum DKK1 protein levels in a total of 241 serum samples from HBV carriers, cirrhotic patients and patients with early and advanced HCC. There was also a significant reduction of serum DKK1 after HCC treatment. These findings suggest that DKK1 plays a role in the progression of HCC. Indeed, serum DKK1 protein has been reported to be increased in some cancers including gastrointestinal, breast and cervical cancers and multiple myeloma [17,19,25]. In addition, in the present study, DKK1 serum protein levels were significantly reduced after liver resection or treatments for HCC. Consistent with our findings, Heider *et al.* showed that serum DKK1 protein decreased in patients with multiple myeloma after anti-myeloma treatment [16]. These data and the current data indicate that elevated serum DKK1 protein is mainly secreted by tumor masses. It is clear that serum DKK1 was upregulated in patients during hepatocarcinogenesis indicating that DKK1 is a new potential serum marker for detecting tumor development and recurrence for HCC.

The long-term survival of HCC patients after surgical resection is unsatisfactory, mainly due to a high incidence of tumor recurrence. Therefore, establishment of some reliable biomarkers for predicting recurrence is particularly important in the clinical management of HCC. With regard to serum tumor biomarker, AFP is most commonly used. However, it has limitations in specificity and sensitivity in the detection of HCC or its recurrence after treatment. There is a need to develop better or complementary serum tumor markers. Currently, the use of a few markers, including Des-gamma-carboxyprothrombin (DCP), glypican-3, α-fucosidase (AFU) and transforming growth factor-beta 1 (TGF-β1) and melanoma-associated antigen gene (MAGE) [26,27], is being investigated on a wider scale. Based on our findings of increased serum DKK1 protein levels in HCC and especially its stepwise increase from cirrhosis via early HCC to advanced HCC and decrease after HCC treatment, it would be valuable to further investigate the value of DKK1 as a tumor biomarker in the diagnosis of early HCC and tumor recurrence.

In this study, it was found that increased serum protein of DKK1 is an unfavorable prognostic marker and was significantly associated with shorter disease-free survival of HCC patients. In HCC patients, disease-free survival is mainly attributed to intrahepatic metastasis and tumor recurrence, both of which are related to venous invasion [28]. Our *in vitro* data have shown that, mechanistically, DKK1 enhanced both cell migration and invasion in HCC cells. Similar observation has also been reported in HEK-293 cell line [29]. From these data, it is clear that DKK1 may be used in promoting intrahepatic metastasis and tumor recurrence. To this end, developing anti-DKK1 monoclonal antibody against serum and tissue DKK1 may be a potentially novel therapeutic means for preventing tumor metastasis and reducing tumor recurrence. In a very recent report, Sato et al. demonstrated that anti-DKK1 monoclonal antibody was able to suppress cell invasion and growth of lung cancer cell line A549 [19]. It supports the use of anti-DKK1 monoclonal antibody to prevent HCC metastasis and recurrence.

From clincopathological analysis, it was found that high levels of serum DKK1 protein significantly correlated with a larger tumor size (Table 2). Therefore, it was hypothesized that DKK1 played a role in promoting cell growth. However, ectopic expression of DKK1 in PLC/PRF/5, the HCC cell line with the lowest level of DKK1 expression, did not alter the cell proliferation rate *in vitro,* either by cell counting assay or MTT assay. Similarly, another HCC cell line, BEL-7402, and a normal liver cell line, MIHA, showed no significant alteration in cell proliferation rates after ectopic expression of DKK1 (data not shown). Interesting, DKK1 enhanced the tumor formation efficiency and promoted tumor growth in nude mice injection assay. The discrepant results between our *in vitro* and *in vivo* data imply that DKK1 may modulate the tumor microenvironment to enhance tumor growth. In fact, DKK1 has been found to be a mediator in endothelial cell activation by suppressing WntB/-catenin pathway [30]. Using *in vivo* microvascular remodeling animal model, Glaw et al. showed that DKK1 significantly increased vascular density and vessel diameter of adult rats [31]. These reports indicate that DKK1 may play a role in microvascular remodeling and activation of angiogenesis and may account for the promotion of *in vivo* tumor growth by DKK1.

It was found that a significant correlation between DKK1 transcript levels and (B-catenin cytoplasmic overexpression with or without nuclear translocation in human HCCs. It has been suggested that DKK1 is a downstream target of Wnt/β-catenin signaling and is regulated under a negative feedback loop in the Wnt/β-catenin signaling [23,24]. Therefore, DKK1 overexpression may be result of hyperactivation of Wnt/β-catenin signaling. However, a subset of HCC cases with high expression of DKK1 transcript but having no or insignificant staining of β-catenin was observed. It indicates that, besides Wnt/β-catenin signaling, other signaling pathway(s) may also contribute to or regulate DKK1 expression. Further investigation on the mechanistic control and regulation of DKK1 in HCC is awaited.

Both serum and tissue DKK1 levels increased in a stepwise manner during the multistep hepatocarcinogenesis. DKK1 enhanced both migratory and invasive abilities of HCC cells *in vitro* and promote tumor growth *in vivo.* Serum DKK1 levels had prognostic significance in HCC. Serum DKK1 is a potential tumor marker with prognostic value for HCC.

A list of references follow, which refer to reference numbers and citations in the text.

### Reference

1. Parkin DM, Bray F, Ferlay J, Pisani P (2005) Global cancer statistics, 2002. CA Cancer J Clin 55: 74-108.
2. Breuhahn K, Longerich T, Schirmacher P (2006) Dysregulation of growth factor signaling in human hepatocellular carcinoma. Oncogene 25: 3787-3800.
3. Greten TF, Korangy F, Manns MP, Malek NP (2009) Molecular therapy for the treatment of hepatocellular carcinoma. Br J Cancer 100: 19-23.
4. Glinka A, Wu W, Delius H, Monaghan AP, Blumenstock C, et al. (1998) Dickkopf-1 is a member of a new family of secreted proteins and functions in head induction. Nature 391: 357-362.
5. Fedi P, Bafico A, Nieto Soria A, Burgess WH, Miki T, et al. (1999) Isolation and biochemical characterization of the human Dkk-1 homologue, a novel inhibitor of mammalian Wnt signaling. J Biol Chem 274: 19465-19472.
6. Semenov MV, Tamai K, Brott BK, Kuhl M, Sokol S, et al. (2001) Head inducer Dickkopf-1 is a ligand for Wnt coreceptor LRP6. Curr Biol 11: 951-961.
7. Mao B, Wu W, Li Y, Hoppe D, Stannek P, et al. (2001) LDL-receptor-related protein 6 is a receptor for Dickkopf proteins. Nature 411: 321-325.
8. Bafico A, Liu G, Yaniv A, Gazit A, Aaronson SA (2001) Novel mechanism of Wnt signalling inhibition mediated by Dickkopf-1 interaction with LRP6/Arrow. Nat Cell Biol 3: 683-686.
9. Kuphal S, Lodermeyer S, Bataille F, Schuierer M, Hoang BH, et al. (2006) Expression of Dickkopf genes is strongly reduced in malignant melanoma. Oncogene 25: 5027-5036.
10. Vibhakar R, Foltz G, Yoon JG, Field L, Lee H, et al. (2007) Dickkopf-1 is an epigenetically silenced candidate tumor suppressor gene in medulloblastoma. Neuro Oncol 9: 135-144.
11. Hall CL, Daignault SD, Shah RB, Pienta KJ, Keller ET (2008) Dickkopf-1 expression increases early in prostate cancer development and decreases during progression from primary tumor to metastasis. Prostate 68: 1396-1404.
12. Forget MA, Turcotte S, Beauseigle D, Godin-Ethier J, Pelletier S, et al. (2007) The Wnt pathway regulator DKK1 is preferentially expressed in hormone-resistant breast tumours and in some common cancer types. Br J Cancer 96: 646-653.
13. Yu B, Yang X, Xu Y, Yao G, Shu H, et al. (2009) Elevated expression of DKK1 is associated with cytoplasmic/nuclear beta-catenin accumulation and poor prognosis in hepatocellular carcinomas. J Hepatol 50: 948-957.
14. Shizhuo W, Tao J, Shulan Z, Bing Z (2009) The expression and significance of Dickkopf-1 in epithelial ovarian carcinoma. Int J Biol Markers 24: 165-170.
15. Sheng SL, Huang G, Yu B, Qin WX (2009) Clinical significance and prognostic value of serum Dickkopf-1 concentrations in patients with lung cancer. Clin Chem 55: 1656-1664.
16. Heider U, Kaiser M, Mieth M, Lamottke B, Rademacher J, et al. (2009) Serum concentrations of DKK-1 decrease in patients with multiple myeloma responding to anti-myeloma treatment. Eur J Haematol 82: 31-38.
17. Politou MC, Heath DJ, Rahemtulla A, Szydlo R, Anagnostopoulos A, et al. (2006) Serum concentrations of Dickkopf-1 protein are increased in patients with multiple myeloma and reduced after autologous stem cell transplantation. Int J Cancer 119: 1728-1731.
18. Qian J, Xie J, Hong S, Yang J, Zhang L, et al. (2007) Dickkopf-1 (DKK1) is a widely expressed and potent tumor-associated antigen in multiple myeloma. Blood 110: 1587-1594.
19. Sato N, Yamabuki T, Takano A, Koinuma J, Aragaki M, et al. (2010) Wnt Inhibitor Dickkopf-1 as a Target for Passive Cancer Immunotherapy. Cancer Res.
20. Makino T, Yamasaki M, Takemasa I, Takeno A, Nakamura Y, et al. (2009) Dickkopf-1 expression as a marker for predicting clinical outcome in esophageal squamous cell carcinoma. Ann Surg Oncol 16: 2058-2064.
21. Yamabuki T, Takano A, Hayama S, Ishikawa N, Kato T, et al. (2007) Dickkopf-1 as a novel serologic and prognostic biomarker for lung and esophageal carcinomas. Cancer Res 67: 2517-2525.
22. Krupnik VE, Sharp JD, Jiang C, Robison K, Chickering TW, et al. (1999) Functional and structural diversity of the human Dickkopf gene family. Gene 238: 301-313.
23. Niida A, Hiroko T, Kasai M, Furukawa Y, Nakamura Y, et al. (2004) DKK1, a negative regulator of Wnt signaling, is a target of the beta-catenin/TCF pathway. Oncogene 23: 8520-8526.
24. Gonzalez-Sancho JM, Aguilera O, Garcia JM, Pendas-Franco N, Pena C, et al. (2005) The Wnt antagonist DICKKOPF-1 gene is a downstream target of beta-catenin/TCF and is downregulated in human colon cancer. Oncogene 24: 1098-1103.
25. Voorzanger-Rousselot N, Goehrig D, Journe F, Doriath V, Body JJ, et al. (2007) Increased Dickkopf-1 expression in breast cancer bone metastases. Br J Cancer 97: 964-970.
26. Qin LX, Tang ZY (2004) Recent progress in predictive biomarkers for metastatic recurrence of human hepatocellular carcinoma: a review of the literature. J Cancer Res Clin Oncol 130: 497-513.
27. Zhou L, Liu J, Luo F (2006) Serum tumor markers for detection of hepatocellular carcinoma. World J Gastroenterol 12: 1175-1181.
28. RTP, Fan ST, Wong J (2000) Risk factors, prevention, and management of postoperative recurrence after resection of hepatocellular carcinoma. Ann Surg 232: 10-24.
29. Kuang HB, Miao CL, Guo WX, Peng S, Cao YJ, et al. (2009) Dickkopf-1 enhances migration of HEK293 cell by beta-catenin/E-cadherin degradation. Front Biosci 14: 2212-2220.
30. Ueland T, Otterdal K, Lekva T, Halvorsen B, Gabrielsen A, et al. (2009) Dickkopf-1 enhances inflammatory interaction between platelets and endothelial cells and shows increased expression in atherosclerosis. Arterioscler Thromb Vasc Biol 29: 1228-1234.
31. Glaw JT, Skalak TC, Peirce SM (2010) Inhibition of canonical Wnt signaling increases microvascular hemorrhaging and venular remodeling in adult rats. Microcirculation 17: 348-357.

**Table 1. Summary of the clinicopathologic characteristics of the HCC patients.**

| | | Number of patients (%) | |
|---|---|---|---|
| Parameters | | qPCR cohort | Serum cohort |
| Total no. of patients | | 99 | 100 |
| Gender | Male | 76 (77) | 82 (82) |
| | Female | 23 (23) | 18 (18) |
| Age | <60 | 63 (64) | 55 (55) |
| | ≥60 | 36 (36) | 45 (45) |
| Tumor stage (TNM) | Early(I & II) | 36 (36) | 34 (34) |
| | Advanced(III & IV) | 55 (56) | 62 (62) |
| Tumor size (cm) | <5cm | 36 (36) | 44 (44) |
| | ≥5cm | 61 (62) | 54 (54) |
| No. of nodules | 1 | 85 (86) | 57 (57) |
| | ≥2 | 12 (12) | 43 (43) |
| Venous invasion | Absent | 46 (46) | 21 (21) |
| | Present | 51 (52) | 28 (28) |
| Serum AFP level (ng/ml) | <400 | 52 (53) | 61 (61) |
| | ≥400 | 41 (41) | 39 (39) |
| Non-tumorous liver | Non-cirrhotic | 47 (47) | 12 (12) |
| | Cirrhotic | 50 (51) | 73 (73) |
| Serum HBsAg | Positive | 72 (73) | 81 (81) |
| | Negative | 22 (22) | 13 (13) |

**Table 2. Clinicopathologic correlation of DKK1 serum levels in HCC patients.**

| | | HCC patients' serum cohort | | |
|---|---|---|---|---|
| Parameters | | DKK1 <1500 pg/ml | DKK1 ≥1500 pg/ml | *P*-value |
| Gender | Male | 63 | 19 | 0.931 |
| | Female | 14 | 4 | |
| Age | <60 | 46 | 9 | 0.081 |
| | ≥60 | 31 | 14 | |
| Tumor stage | Early (I & II) | 30 | 4 | 0.075 |
| (TNM) | Advanced (III & IV) | 44 | 18 | |
| Tumor size | <5cm | 39 | 5 | 0.027* |
| (cm) | ≥5cm | 37 | 17 | |
| No. of nodules | 1 | 46 | 11 | 0.311 |
| | ≥2 | 31 | 12 | |
| Venous invasion | Absent | 15 | 6 | 0.739 |
| | Present | 22 | 6 | |
| Serum AFP level | <400 | 48 | 13 | 0.808 |
| (ng/ml) | ≥400 | 29 | 10 | |
| Non-tumorous liver | Non-cirrhotic | 7 | 5 | 0.119 |
| | Cirrhotic | 60 | 13 | |
| Serum HBsAg | Positive | 62 | 19 | 1.000 |
| | Negative | 10 | 3 | |

| | | | | |
|---|---|---|---|---|
| * *P* <0.05 | | | | |

## Claims

1. An anti-DKK1 monoclonal antibody for use in a method of treating or preventing DKK1-mediated hepatocellular carcinoma the method comprising administering to a subject having hepatocellular carcinoma and (a) high levels of DKK1 protein in serum of the subject compared to subjects without hepatocellular cancer, or (b) overexpression of DKK1 in hepatocellular carcinoma tissue of the subject compared to non-tumorous hepatocellular tissue of the subject an effective amount of an anti-DKK1 monoclonal antibody in a pharmaceutical formulation.

2. The antibody for use in a method of claim 1, wherein the antibody is used to inhibit the DKK1-mediated cell migration, invasion, tumor growth and cancer metastasis in liver cancer and/or reduces recurrence of liver cancer.

3. The antibody for use in a method of claim 1 or 2 wherein the monoclonal antibody is a humanized monoclonal antibody.

## Patentansprüche

1. Ein anti-DKK1 monoklonaler Antikörper zur Verwendung in einem Verfahren zur Behandlung oder zur Verhütung eines DKK1-vermittelten Leberzellkarzinoms, umfassend die Verabreichung einer wirksamen Menge eines anti-DKK1 monoklonalen Antikörpers in einer pharmazeutischen Formulierung an ein Subjekt mit einem Leberzellkarzinom und mit (a) hohem Dkkl Proteingehalt im Serum im Vergleich zu Subjekten, die kein Leberzellkarzinom aufweisen, oder (b) mit Überexpression von DKK1 im Leberzellkarzinom-Gewebe im Vergleich zu dem nicht-tumorösen Leberzellkarzinom-Gewebe des Subjekts.

2. Der Antikörper zur Verwendung in einem Verfahren nach Anspruch 1, wobei der Antikörper verwendet wird, um die/das DKK1-vermittelte Zell-Migration, -Invasion, Tumorwachstum und Krebsmetastase bei Leberkrebs zu blockieren, und/oder um das Wiederauftreten von Leberkrebs zu reduzieren.

3. Der Antikörper zur Verwendung nach Anspruch 1 oder 2, wobei der monoklonale Antikörper ein humanisierter monoklonaler Antikörper ist.

## Revendications

1. Anticorps monoclonal anti-DKK1 pour une utilisation dans un procédé de traitement ou de prévention du carcinome hépatocellulaire induit par DKK1, le procédé comprenant l'administration à un sujet ayant un carcinome hépatocellulaire et (a) des quantités élevées de protéine DKK1 dans le sérum du sujet par rapport aux sujets exempts de cancer hépatocellulaire, ou (b) une surexpression de DKK1 dans les tissus du carcinome hépatocellulaire du sujet par rapport aux tissus hépatocellulaires non tumoraux du sujet, d'une quantité efficace d'un anticorps monoclonal anti-DKK1 dans une formulation pharmaceutique.

2. Anticorps pour une utilisation dans un procédé selon la revendication 1, dans lequel l'anticorps est utilisé pour inhiber la migration cellulaire induite par DKK1, l'invasion, la croissance de la tumeur et les métastases cancéreuses dans le cancer du foie et/ou réduit la récidive du cancer du foie.

3. Anticorps pour une utilisation dans un procédé selon la revendication 1 ou 2, dans lequel l'anticorps monoclonal est un anticorps monoclonal humanisé.
